# EUROPEAN PATENT APPLICATION

(11) **EP 3 662 877 A1**
(43) Date of publication of application: **10.06.2020**
(21) Application number: 19212950.0
(22) Date of filing: 02.12.2019
(51) Int. Cl.: A61F 13/06, A41D 13/06

(54) **heel protector**

(30) Priority: 03.12.2018 ES 201831868 U
(71) Applicant: Compte Lopez, Josefa, 08560 Manlleu (Barcelona) (ES)
(72) Inventor: Compte Lopez, Josefa, 08560 Manlleu (Barcelona) (ES)
(74) Representative: Durán-Corretjer, S.L.P.

(57) **Abstract**

Heel protector for prevention of foot decubitus ulcers comprising a first layer of textile fibre intended to be in contact with the skin of the foot; a second layer for passive cushioning arranged in the heel area; a third layer of silicone suitable for being used in orthosis intended to cover at least the heel area and malleoli and a fourth layer of textile fibre covering the previous layers.

## Description

The present invention relates to a novel heel protector. In particular, the present invention relates to a novel heel protector which prevents decubitus ulcers.

Decubitus ulcers, also known as pressure ulcers, are skin and underlying tissue injures that can be produced as a result of a prolonged pressure on the skin between two hard surfaces, the bones of the affected person and the support (bed, chair, etc.), causing tissue ischemia first and subsequent tissue necrosis. Commonly, decubitus ulcers or pressure ulcers occur in skin covering bony parts of the body, such as heels, ankles, hips, coccyx, etc.

Apart from prolonged pressure on the skin, another factor that can cause pressure ulcers is the friction between the skin and the clothes and/or the bed linen, because such friction produces a force parallel to the skin, which causes dermal tissue erosion. A third reason that may cause pressure ulcers is the shearing that occurs when two surfaces, in this case the skin and clothes and/or bed linen, move in opposite directions.

People with a higher risk for suffering decubitus ulcers or pressure ulcers are those who, owing to either a disease or any other reason, have limited mobility that limits or hinders the possibility of changing their body posture and/or the ones who spend most of their time in bed or on a chair. Among these groups of people with a high risk for suffering pressure ulcers, elderly people stand out, but also at risk are patients on palliative care, with spinal cord injuries, with obesity problems and patients who need a resting period after, for example, a surgical intervention.

Measures to prevent and/or mitigate the development of pressure ulcers can be divided into those intrinsic to the patient and those that are extrinsic. Intrinsic measures include keeping the skin clean, hydrated, though avoiding excessive moisture. As for extrinsic measures, there are general and local ones. Among the general measures the use of Pressure-Relieving Support Surfaces (PRSS) stands out. Among the local ones, the protections whose objective is to reduce pressure in specific parts of the patient's body stand out. Said local protections are mainly used on the heels and are therefore known as heel protectors.

Traditionally, padded heel bandages have been used, but are currently not advisable, since it is necessary to examine the ulcer area at least once a day and this implies removing and subsequently reposition such bandage every day, which requires a considerable amount of time.

Among the most used heel protectors over the past years, the four following models stand out:
- Natural skin heel protector. This type is recommended to prevent pressure ulcer formations in bedridden patients. These heel protectors are usually made of lambskin, which provides heat in winter and serves as thermal insulation in summer. They protect the malleoli and the heel and are fastened with a closure located in the dorsomedial area of the foot.
- Hydrocellular, non-adhesive, sterile and multi-layer dressings. Indicated for the prevention of pressure ulcers on heels and elbows. They are flexible and have a great capacity to reduce the pressure on bony prominences, protect the malleoli and have the capacity to absorb wound exudate. They are fastened with and adhesive cushioned strap. They are marketed under Askina® Heel brand by B. Braun Melsungen AG.
- Polyester heel protector. These are heel protectors for the prevention of pressure ulcers which, as their name suggests, are made of 100% polyester. They usually have a double fastening system located in the dorsomedial area of the foot.
- Hydrocellular non-adhesive dressings indicated for the treatment of exuding heel wounds. They are made of breathable material, require a secondary attaching system and contribute to pressure reduction. They are marketed under Allevyn® heel brand by T J Smith & Nephew Limited.

It is widely known by professionals that, although none of these heel protectors are difficult to place, they do pose, to a greater or lesser extent, some problems with regard to their fastening to the foot. These poor fastening can result in the formation of oedemas in the malleoli or other parts of the foot.

Another problem with state-of-the-art heel pads is that some of them have a very short lifespan, i.e. they wear out very quickly, making it necessary to replace them relatively often, with the economic cost that this represents.

In general, hydrocellular heel protectors are not considered by health professionals, as an efficient way to protect malleolus.

The general opinion of health professionals regarding their overall satisfaction with the existing heel pads, i.e. those belonging to the state of the art, is that none offers complete satisfaction, to the extent that some obtain a really low rating.

It is an object of the present invention to disclose a heel protector which prevents pressure ulcers or decubitus ulcers and successfully solves all the problems mentioned above, which are common in the existing heel protectors. For this purpose, the present invention discloses a heel protector for prevention of foot decubitus ulcers, which comprises:
- A first layer of textile fibre intended to be in contact with the skin of the foot.
- A second layer for passive cushioning arranged in the heel area.
- A third layer of silicone suitable for being used in orthosis intended to cover at least the heel area and malleoli.
- A fourth layer of textile fibre covering the previous layers.

Advantageously, said second layer for passive cushioning is placed over the first layer of textile fibre.

Advantageously, said third layer of silicone is placed over the first layer of textile fibre and the second layer for cushioning.

Preferably, the aforementioned layers comprised in the heel protector object of the present invention are attached to the foot by a strap which, passing through the area of the malleoli and the plantar area of the forefoot, intertwines on the back of the foot.

In an embodiment, the heel protector object of the present invention can comprise a layer of a stretchable material covering the lower layers and keeping them attached to the foot. Said layer of stretchable material can either be the previously mentioned fourth layer or an additional one. Said layer of stretchable material can complement or replace said strap.

Preferably, said strap can have a first and a second end, both being attached to each other by means of non-permanent attaching means. More specifically, the attachment between both ends can be located in the plantar area of the forefoot.

Advantageously, the textile fibre of the first layer can be made of cotton. However, other textile fibres, natural or synthetic, can also be used for the first layer.

Preferably, the layer for passive cushioning can be made of polyurethane. Said polyurethane and can be in the form of foam. More preferably, said layer for passive cushioning can be made of cellular polyurethane. Said cellular polyurethane can be in the form of foam. In particular, this cellular polyurethane can be PORON® marketed by Rogers Corporation.

Preferably, the silicone of the third layer can be a liquid polycondensation silicone. In particular, said liquid polycondensation silicone can be a 11504 silicone. However, it is also possible to use other types of silicone or equivalent materials, being of the type that can adapt to the morphology of the foot and provide a suitable cushioning, as for example, semiliquid silicones.

Advantageously, the textile fibre of the fourth layer can be made of cotton. However, other types of textile fibre, natural or synthetic, can also be used for said fourth layer.

Preferably, the heel protector object of the present invention can be custom-made by a health professional or a user with sufficient knowledge, i.e., said professional or user can make the heel protector by placing the different layers in situ. Alternatively, these heel protectors can be marketed in several sizes and shapes to suit different types of foot.

More specifically, it is also disclosed a method of manufacturing a heel protector configured to prevent pressure ulcers on the user's foot. The method of manufacturing a heel protector comprising the following consecutive steps:
- Place a first layer of textile fibre on the skin of the foot, so that it adjusts to the shape of the foot.
- Place a second layer for passive cushioning on the heel, this second layer being placed over the first one.
- Place a third layer of silicone suitable for being used in orthosis over the first textile fibre layer and the second layer for cushioning, so that it covers, at least, the heal and the malleoli.
- Place a fourth layer of textile fibre covering the previous layers, so that it adjusts to the shape of the foot.

Preferably, the process of manufacturing a heel protector comprises a further step of increasing the fastening of the mentioned layers to the foot by passing a strap over the malleoli and the plantar are of the forefoot, in such a way that they intertwine on the back of the foot, i.e. providing a fastening in the shape of an 8.

In the present document, cotton fabrics are understood as textile fibres composed solely of cotton, as well as textile fabrics partially made of cotton, i.e. comprising a combination of cotton with at least another material.

A series of drawings representing one embodiment of the heel protector object of the present invention are appended to ensure better understanding through explanatory but not limiting examples.
- Figure 1 shows in a profile view the first and second layer of an exemplary embodiment of a heel protector according to the present invention.
- Figure 2 shows in a profile view the third layer of an exemplary embodiment of a heel protector according to the present invention.
- Figure 3 shows in a profile view the fourth layer of an exemplary embodiment of a heel protector according to the present invention.
- Figure 4 shows in a perspective view a fastening strap of an exemplary embodiment of a heel protector according to the present invention.
- Figure 5 shows an elevation view of an exemplary embodiment of a heel protector according to the present invention.

In the figures, identical or equivalent elements have been given the same reference numerals.

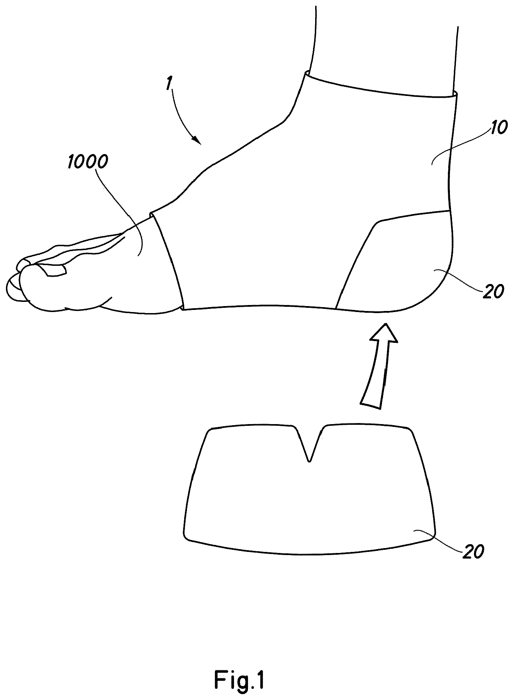

Figure 1 shows the first and second layer (10, 20) of an exemplary embodiment of a heel protector (1) according to the present invention. Said first layer (10) adjusts to the shape of the foot (1000) of the heel protector user and provides a great absorption capacity in case of sweating, thus avoiding a potential maceration of the skin and giving the heel protector a high comfort level. In the exemplary embodiment shown, the first layer (10) of textile fibre is made of cotton. However, in other embodiments it could also be made of other textile fibres, natural or synthetic, that provide a similar, or better, feel and sweat absorption capacity. In addition to the above, this first layer (10) to prevent the subsequent layers from being in direct contact with the skin of the foot (1000) of the patient or user of the heel protector (1), which, if produced, could cause chafing, sweating, etc.

The second layer (20) is made of a technical material for passive cushioning and is responsible for providing a greater amount of cushioning and pressure absorption to the heel and adjacent areas. This second layer (20) is placed on the heel and on top of the first textile layer (10). This second layer (20) can either cover just the heel or extend to the adjacent areas.

In the exemplary embodiment shown, the second layer (20) is made of cellular polyurethane, and more specifically, of PORON®. Within the range of cellular polyurethane that PORON® offers, any one suitable for use in prosthesis and orthosis can be used. Although the second layer (20) of a heel protector (1) according to the present invention is preferably made of cellular polyurethane, it can also be made of other materials with similar properties.

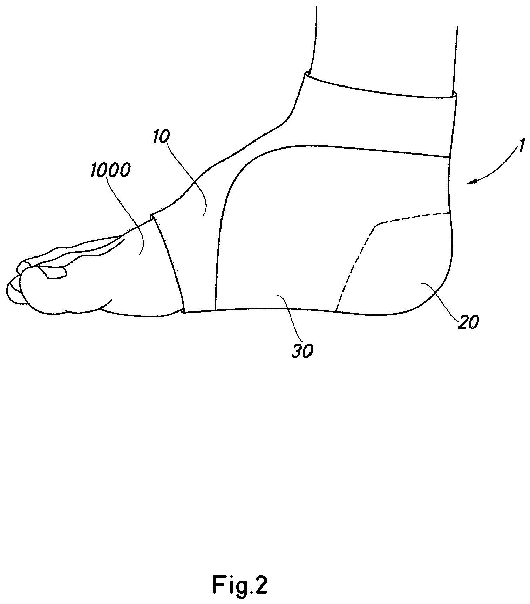

Figure 2 shows how the third layer (30) is placed over the first and second layers (10, 20). Preferably, the third layer is made of silicone suitable for use in orthosis. Said silicone has, preferably, a great adaptability and malleability that makes it easy to spread over the area to be covered, in this case, the heel and malleoli, thus providing a pressure relief both in the heel and in the malleoli. In the exemplary embodiment shown, the silicone of the third layer (30) is a liquid polycondensation silicone of the type 11504, although other types of silicones or similar materials can also be used.

As can be seen, said third layer (30) of silicone is not directly in contact with the foot (1000) of the patient or user of the heel protector (1), since this third layer (30), like the second layer (20), it is placed over the first layer (10) without going beyond it.

Although Preferably the heel protector (1) object of the present invention comprises the third layer (30) of silicone since in this way the prevention of the formation of pressure ulcers is maximized, in cases where the user of the heel protector (1) or patient already suffers from pressure ulcers in the heel or surroundings, part of the silicone from the third layer (30) can be removed so that the pressure in the area of the already existing ulcer is reduced and, consequently, the healing time of the eschar or ulcer is shortened. If this type of intervention is necessary, the silicone is preferably removed from the area over the existing ulcer. In extreme cases, the whole third silicone layer (30) can be removed, in which case the heel protector would comprise the layers (10, 20 and 40). If the silicone of the third layer (30) is partially or totally removed, it can be put back to the heel protector once the wound or ulcer has healed.

In figure 2 the second layer (20) has been represented with dashed lines in order to represent that is hidden under the third layer (30).

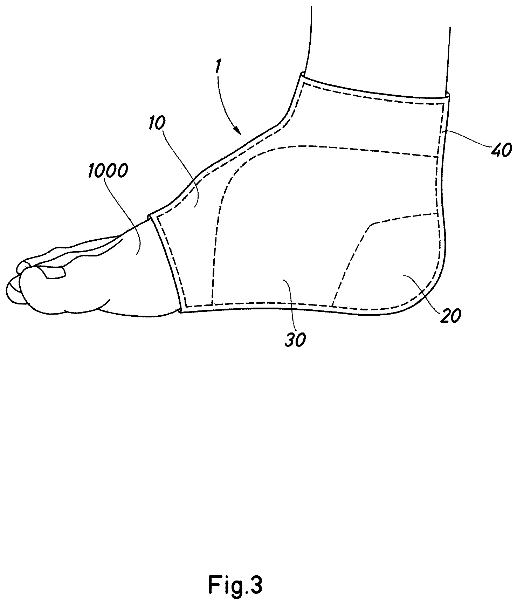

Figure 3 shows how the first, second and third layers (10, 20, 30) of the heel protector (1), which are represented in dashed lines, are covered by a fourth layer (40). Said fourth layer (40) is made of textile fibre and is arranged in such a way that it fits the shape of the foot (1000) of the patient or user of the heel protector (1).

In the exemplary embodiment shown, the fourth layer (40) is made of cotton, which facilitates the sliding, for example, between the heel protector (1) and the bed sheets, and thus avoiding the risk of injury or ulcers in the foot (1000) due to the rubbing of the heel protector (1) and the surface on which rests. In other embodiments, said fourth layer (40) can be made of textile fibres other than cotton, either natural or synthetic.

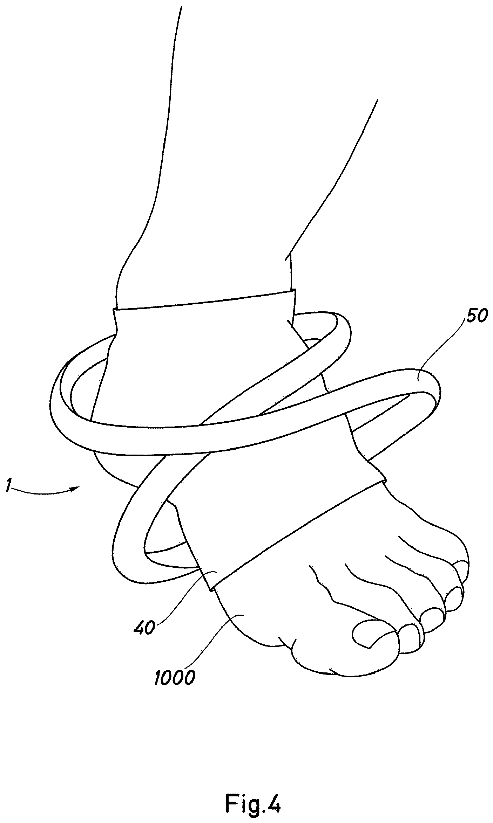

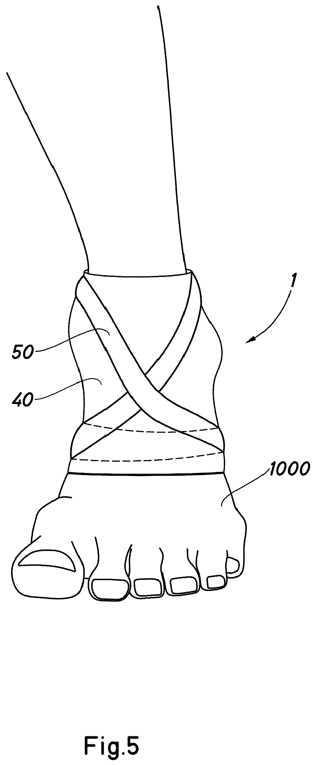

Figures 4 and 5 show how the exemplary embodiment shown comprises a strap (50) for fastening to the foot (1000) the different layers (10, 20, 30, 40) comprised in the heel protector (1). As can be seen, said strap (50) provides a fastening in the shape of an 8, that is, said strap (50) goes over the malleoli and the plantar area of the forefoot and intertwines at the back of the foot (1000). This type of fastening in the shape of an 8, by not making a circular closure, aids the venous return, that is, the blood circulation, while providing stability to the heel protector (1) and prevents movement between the different layers (10,20,30,40) comprised in the heel protector (1).

In the exemplary embodiment shown, both ends of the strap (50) are joined together by non-permanent attaching means, and more specifically, by a closure system that comprises two straps made of different fabrics which attach to each other when they come into contact, usually marketed under the Velcro® brand. In other embodiments, this union between both ends of the strap (50) can be made, for example, with safety pins, surgical tape, adhesive tape, etc.

In other embodiments, said strap (50) can be replaced by a layer made of a stretchable material that fits the foot and keeps the inner layers in place and tightened to it. Said layer of an stretchable material can be a fifth layer over the fourth layer (40), or a fourth layer that in addition to, for example, cotton, also comprises some stretchable textile fibres, so that the fourth layer (40) is capable of attaching the first, second and third layers (10, 20, 30) of the heel protector (1).

Although the heel protector object of the present invention is preferably manufactured individually and in situ for each user by a duly qualified professional, it can also be sold in packs that contain heel protectors in different sizes and shapes in order to be able to adapt to different sizes and shapes of feet.

Although the invention has been set out and described with reference to embodiments thereof, it should be understood that these do not limit the invention, and that it is possible to alter many structural or other details that may prove obvious to persons skilled in the art after interpreting the subject matter disclosed in the present description, claims and drawings. In particular, in principle and unless otherwise explicitly stated, all the features of each of the different embodiments and alternatives shown and/or suggested can be combined. Therefore, the scope of the present invention includes any variant or equivalent that could be considered covered by the broadest scope of the following claims.

## Claims

1. Heel protector for prevention of foot decubitus ulcers, **characterised in that** it comprises:
- A first layer of textile fibre intended to be in contact with the skin of the foot.
- A second layer for passive cushioning arranged in the heel area.
- A third layer of silicone suitable for being used in orthosis intended to cover at least the heel area and malleoli.
- A fourth layer of textile fibre covering the previous layers.

2. Heel protector, according to claim 1, **characterised in that** said second layer for passive cushioning is placed over the first layer of textile fibre.

3. Heel protector, according to claim 1 or 2, **characterised in that** said third layer of silicone is placed over the first layer of textile fibre and the second layer for cushioning.

4. Heel protector, according to any one of the preceding claims, **characterised in that** the aforementioned layers are attached to the foot by a strap which, passing through the area of the malleoli and the plantar area of the forefoot, intertwines on the back of the foot.

5. Heel protector, according to claim 4, **characterised in that** said strap has a first and a second end, both being attached to each other by means of non-permanent attaching means.

6. Heel protector, according to claim 5, **characterised in that** in which the attachment between both ends is in the plantar area of the forefoot.

7. Heel protector, according to any one of the preceding claims, **characterised in that** the textile fibre of the first layer is made of cotton.

8. Heel protector, according to any of the preceding claims, **characterised in that** the layer for passive cushioning is made of polyurethane.

9. Heel protector, according to claim 8, **characterised in that** said layer for passive cushioning is made of cellular polyurethane.

10. Heel protector, according to any one of the preceding claims, **characterised in that** the silicone of the third layer is a liquid polycondensation silicone.

11. Heel protector, according to any one of the preceding claims, **characterised in that** said liquid polycondensation silicone is silicone type 11504.

12. Heel protector, according to any one of the preceding claims, **characterised in that** the textile fibre of the fourth layer is made of cotton.
